# EUROPÄISCHE PATENTSCHRIFT

(11) **EP 0 173 199 B1**
(45) Veröffentlichungstag und Bekanntmachung des Hinweises auf die Patenterteilung: **13.07.1994**
(21) Anmeldenummer: 85110342.4
(22) Anmeldetag: 19.08.1985
(51) Int. Cl.: C07D 209/02, A61K 31/40

(54) **cis, endo-2-Azabicycloalkan-3-carbonsäure-Derivate, Verfahren zu deren Herstellung, deren Verwendung sowie Zwischenprodukte bei deren Herstellung**
Cis, endo-2-azabicycloalkane-3-carboxylic-acid derivatives, processes for their manufacture, their use and intermediate products obtained in their manufacture
Dérivés de l'acide cis, endo-aza-2 bicycloalcane carboxylique-3, procédés pour leur préparation et les produits intermédiaires lors de leur préparation

(30) Priorität: 28.08.1984 DE 3431541
(43) Veröffentlichungstag der Anmeldung: 05.03.1986
(73) Patentinhaber: HOECHST AKTIENGESELLSCHAFT, 65926 Frankfurt am Main (DE)
(72) Erfinder: Urbach, Hansjörg, Dr., D-6242 Kronberg/Taunus (DE); Henning, Rainer, Dr., D-6234 Hattersheim am Main (DE); Hertzsch, Winfried, Dr., D-6233 Kelkheim (Taunus) (DE)

(56) Entgegenhaltungen:
- EP-A- 0 079 022
- WO-A-86/00896
- JOURNAL FUER PRAKTISCHE CHEMIE, Band 314, 1972, Leipzig. S. Hauptmann et al. "Beiträge zum Raktionsverhalten von 2-Amino-vinylcarbonylverbindungen" Seiten 353-364

## Beschreibung

Die Erfindung betrifft Verbindungen der Formel I 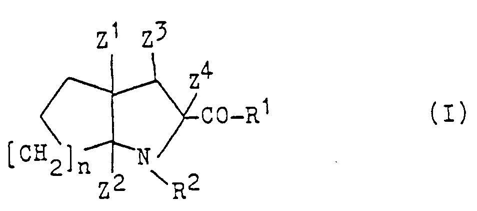
in welcher
- n=: 1, 2 oder 3 ist und

a)
   - Z¹: und Z² sowie Z³ und Z⁴ jeweils zusammen für eine chemische Bindung stehen,
   - R¹: (C₁-C₆)-Alkoxy, (C₆-C₁₂)-Aryl-(C₁-C₄)-alkoxy, Amino, (C₁-C₆)-Alkylamino oder Di-(C₁-C₆)-alkylamino und
   - R²: Wasserstoff, (C₁-C₈)-Alkyl, (C₆-C₁₂)-Aryl, (C₆-C₁₂)-Aryl-(C₁-C₆)-alkyl, p-Nitrobenzyl, p-Methoxybenzyl, (C₃-C₈)-Cycloalkyl oder (C₇-C₁₂)-Alkylcycloalkyl bedeuten wobei die aus J. prakt. Chem. 314 [1972] 353, 354 bekannten Verbindungen der Formel I ausgenommen sind, in denen Z¹ - Z⁴ wie unter a) definiert sind,
n= 1, 2 oder 3; R¹ Ethoxy und R² Methyl bedeuten.

Alkyl (als solches oder als Bestandteil anderer Reste) kann geradkettig oder verzweigt sein. Unter Aryl wird hier und im folgenden vorzugsweise Phenyl, Naphthyl oder Biphenylyl, insbesondere aber Phenyl verstanden.

Aus der EP-A-79022 sind Verbindungen der Formel IV und deren Spiegelbild, 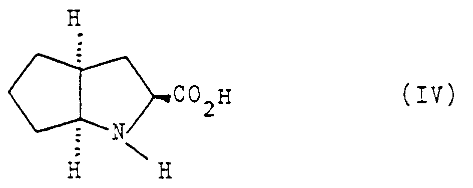
in welcher die Wasserstoffe an den Brückenkopf-C-Atomen zueinander cis-konfiguriert sind, die Carboxygruppe am C-Atom 3 endoständig zum bicyclischen Ringsystem orientiert ist, sowie ein Verfahren zu deren Herstellung durch Umsetzung eines Enamins des Cyclopentanons mit einer Verbindung der Formel V 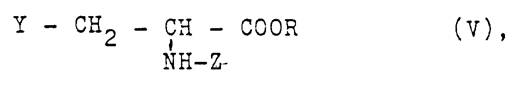
in welcher R Alkyl oder Aralkyl, Y eine nucleofuge Gruppe und Z Alkanoyl, Aroyl oder eine andere, in der Peptidchemie übliche Schutzgruppe bedeuten,
Cyclisierung der erhaltenen Verbindungen der Formel VI, 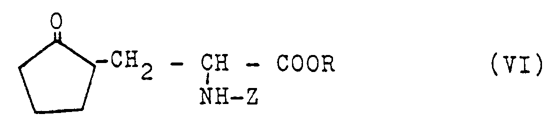
zu Verbindungen der Formel VII a bzw. VII b, 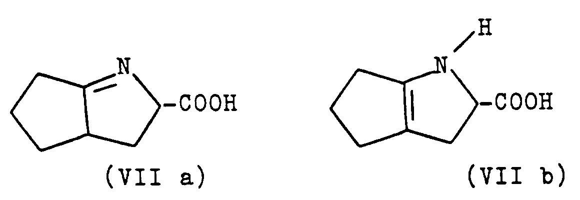
und anschließende Hydrierung dieser Verbindungen bekannt (siehe Reaktionsschema I).

### Reaktionsschema I (Ausgangsmaterialien im Handel erhältlich):

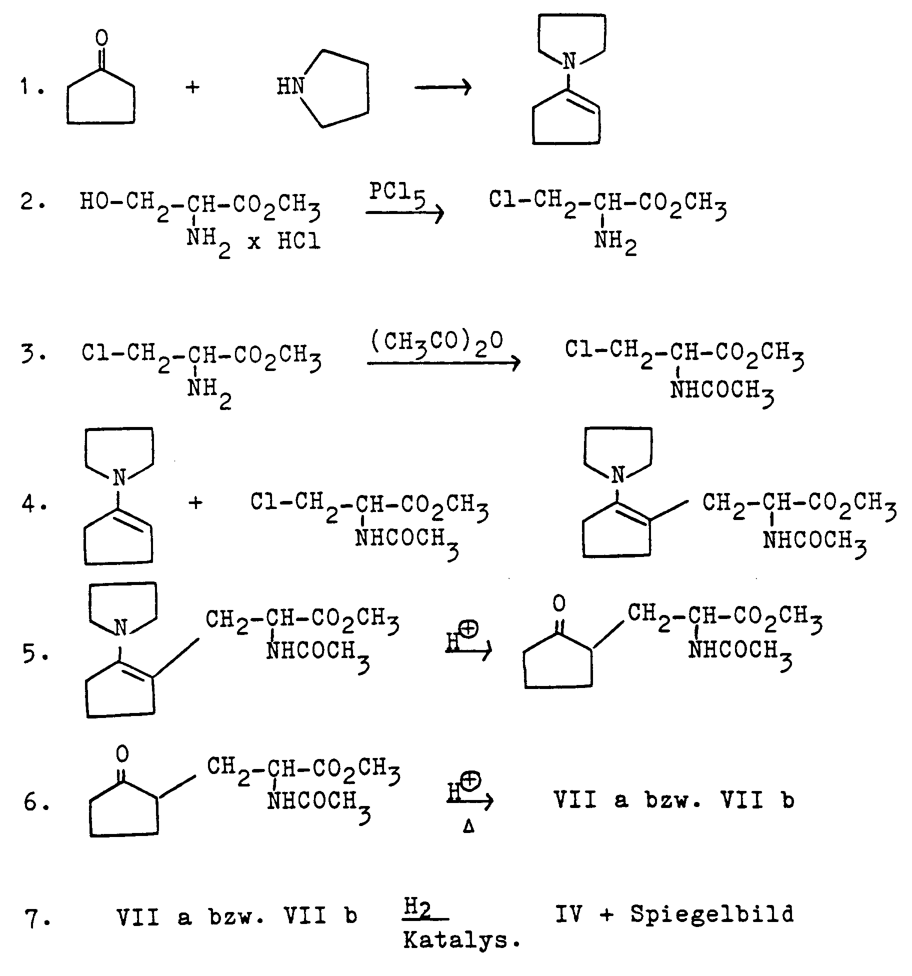

Geht man von käuflichen Ausgangsmateralien aus (Cyclopentanon und D,L-Serinmethylesterhydrochlorid), erhält man die Verbindung der Formel IV + Spiegelbild in einer 7-stufigen Synthese. Da die Verbindungen der Formel IV Zwischenprodukte bei der Herstellung von hochaktiven Angiotensin-Converting-Enzyme-Inhibitoren darstellen, wie sie aus der EP-A-79022 bekannt sind, ist es von großem Interesse, Verbindungen der Formel IV oder verwandte Verbindungen besonders wirtschaftlich, d.h. bei Einsatz von kostengünstigen Ausgangsmaterialien in möglichst geringen Reaktionschritten, herzustellen.

Die erfindungsgemäßen Verbindungen der Formel I sind auf einfache Weise zugängliche Zwischenprodukte bei der Herstellung hochaktiver Angiotensin-Converting-Enzyme-Inhibitoren.

Bevorzugt sind Verbindungen der Formel I, in der
R¹ (C₁-C₆)-Alkoxy und
R² Wasserstoff oder (C₆-C₁₂)-Aryl-(C₁-C₆)-alkyl p-Nitrobenzyl oder p-Methoxybenzyl bedeuten, dabei sind insbesordere solche (C₆-C₁₂)-Aryl-(C₁-C₆)-alkylreste bevorzugt, die katalytisch abhydrierbar sind, wie z.B. Benzyl, p-Nitrobenzyl, p-Methoxybenzyl, α-Phenyl-ethyl oder Benzhydryl.

Verbindungen der Formel I, in der Z¹ - Z⁴ wie oben unter a) definiert sind, weisen 2 Doppelbindungen auf (s. Formel VIII). 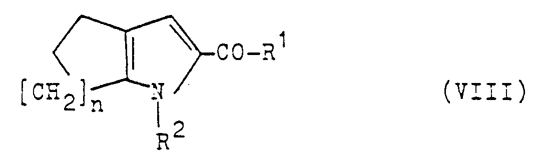
Verbindungen der Formel I, in der Z¹ - Z⁴ jeweils für Wasserstoff stehen, besitzen zwei zueinander cis-konfigurierte Wasserstoffatome an den Brückenkopfatomen 1 und 5, und die CO-R¹-Gruppe ist endoständig zum bicyclischen Ringsystem orientiert. Das Kohlenstoffatom in Position 3 ist R-konfiguriert (Formel IX) oder S-konfiguriert (Formel X). Die vorliegende Erfindung umfaßt sowohl optisch reine Verbindungen der Formeln IX und X als auch deren Gemisch. 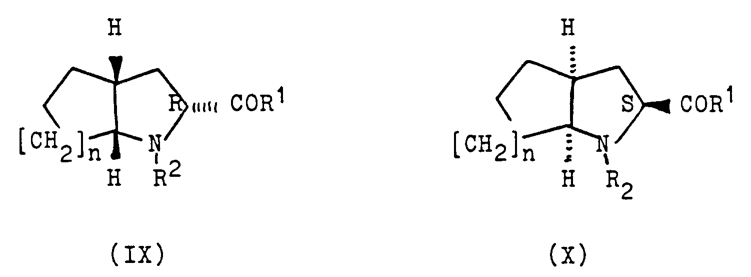
Bevorzugt ist die S-Konfiguration in Position 3.

Weiterhin sind Verbindungen der Formel I bevorzugt, in denen n= 1 bedeutet, insbesondere solche Verbindungen der Formel VIII in denen R¹ Ethoxy und R² Benzyl bedeutet.

Die Erfindung betrifft ferner ein Verfahren zur Herstellung einer Verbindung der Formel I, in welcher
n= 1, 2 oder 3 ist,
Z¹, Z², Z³ und Z⁴ jeweils für Wasserstoff stehen, wobei Z¹ und Z² zueinander cis-konfiguriert sind und die COR¹-Gruppe am C-Atom 3 endoständig zum bicyclischen Ringsystem orientiert ist,
R¹ (C₁-C₆)-Alkoxy, (C₆-C₁₂)-Aryl-(C₁-C₄)-alkoxy, Amino, (C₁-C₆)-Alkylamino oder Di-(C₁-C₆)-alkylamino und
R² Wasserstoff, (C₁-C₈)-Alkyl, (C₆-C₁₂)-Aryl, (C₆-C₁₂)-Aryl-(C₁-C₆)-alkyl, (C₆-C₈)-Cycloalkyl oder (C₇-C₁₂)-Alkylcycloalkyl bedeuten, das dadurch gekennzeichnet ist, daß man eine Verbindung der Formel I, in welcher
Z¹ und Z² sowie Z³ und Z⁴ jeweils zusammen für eine chemische Bindung stehen und
n, R¹ und R² wie oben definiert sind,
katalytisch hydriert,
sowie ein Verfahren zur Herstellung einer Verbindung der Formel I, in der Z¹, Z², Z³, Z⁴, R¹ und R² wie eingangs unter a) definiert ist und n obige Bedeutungen hat, das dadurch gekennzeichnet ist, daß man ein Glycinderivat der Formel II

R²-NH-CH₂-CO-R¹ (II)

in welcher R¹ und R² wie oben definiert sind, umsetzt mit einer Verbindung der Formel III 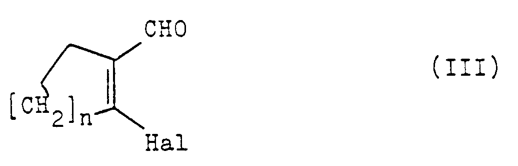
in welcher n= 1, 2 oder 3 ist und Hal für Halogen, vorzugsweise Chlor, steht.

Vorzugsweise stellt man eine Verbindung der Formel VIII her, in der n= 1, R¹ Ethoxy und R² Benzyl bedeuten. Diese Verbindung kann dann durch katalytische Hydrierung unter Abspaltung der Benzylgruppe in eine Verbindung der Formeln IX bzw. X überführt werden, worin n= 1, R¹ Ethoxy und R² Wasserstoff bedeuten.

Mit den erfindungsgemäßen Verfahren ist es gelungen, bei kostengünstigen käuflich erhältlichen Ausgangsmaterialien (z.B. Cyclopentanon und N-Benzylglycin-ethylester) die Anzahl der Reaktionsschritte bei guten Aubeuten auf vier zu reduzieren.

Eine bevorzugte Ausführungsform ist im Reaktionsschema II dargestellt (R¹= OH, R²= H, n= 1, 2 3).

### Reaktionsschema II:

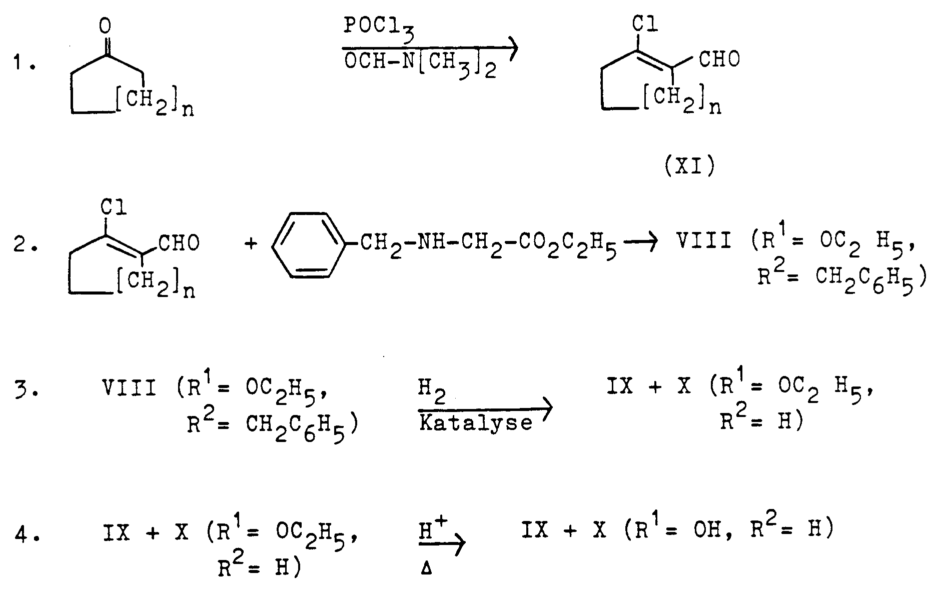

setzt man Glycinderivate der Formel (II) mit R¹ und R² in der oben angegebenen Bedeutung in die 2. Reaktionsstufe ein, so erhält man Verbindungen der Formel VIII, die katalytisch zu Verbindungen der Formel IX und deren Spiegelbild X hydriert werden.

Ein bevorzugtes erfindungsgemäßes Verfahren ist dadurch gekennzeichnet, daß die literaturbekannten Verbindungen der Formel XI mit n= 1, 2, 3 (Chem. Ber., 2743 (1960), die sehr leicht und in sehr guter Ausbeute zugänglich sind, mit Glycinderivaten der Formel II umgesetzt werden. Besonders bevorzugt ist die Umsetzung mit N-Benzylglycinethylester. Die Reaktion kann ohne Lösungsmittel in der Weise durchgeführt werden, daß äquivalente Mengen der Verbindungen der Formel II und XI im Gegenwert einer organischen Base, wie z.B. Triethylamin, Diisopropylethylamin, Dicyclohexylethylamin, N-Ethylmorpholin, etc. in einem Temperaturbereich zwischen 0° und 160°C, Vorzugsweise zwischen 20°C und 120°C, umgesetzt werden: Die Base kann im äquimolaren Verhältnis, im Überschuß oder auch in Unterschuß eingesetzt werden. Die organische Base kann auch ersetzt werden durch einen entsprechenden Überschuß der Verbindung II. Die oben beschriebene Reaktion wird ohne Lösungsmittel oder in einem polaren oder unpolaren organischen Lösungsmittel in einem Temperaturbereich zwischen 0°C und Siedetemperatur des Lösungsmittels durchgeführt. Als besonders vorteilhaft haben sich Toluol oder Benzol erwiesen. Bei Rückflußtemperatur kann mit diesen Lösungsmitteln das entstehende Reaktionswasser herausgeschleppt werden. Das Reaktionswasser läßt sich auch mit anderen wasserbindenden Mitteln, wie z.B. MgSO₄, Molekularsieb, etc. entfernen. Das Reaktionsgemisch wird gegebenenfalls nach Abdampfen der Lösungsmittel mit einem unpolaren aprotischen Lösungsmittel, wie z.B. Hexan, Petrolether, etc. extrahiert, wobei das Pyrrolderivat schon relativ sauber anfällt. Es kann in dieser Reinheit in die weitere Reaktion eingesetzt werden. Ist eine weitere Reinigung erforderlich, kann über Kieselgel oder Aluminiumoxid mit einem organischen Lösungsmittel filtriert werden. Das Pyrrolderivat der Formel VIII wird in einem organischen Lösungsmittel, vorzugsweise in einem Alkohol wie z.B. Ethanol oder Methanol, katalytisch hydriert zu den Verbindungen der allgemeinen Formel IX und deren Spiegelbild X. Die Hydrierung kann bei Raumtemperatur oder erhöhter Temperatur bis zu 100°C unter Normalbedingungen oder unter Druck vorgenommen werden. Bevorzugt wird eine Temperatur zwischen 20° und 60°C und ein Druck, der zwischen 1 und 100 bar liegt. Als Katalysatoren eignen sich Raney-Nickel oder Edelmetallkatalysatoren wie z.B. Palladium, Platin, Rhodium. Als besonders vorteilhaft hat sich Palladium auf Kohle erwiesen. Die Hydrierung kann in Gegenwart einer anorganischen oder organischen Säure erfolgen. Als besonders vorteilhaft hat sich der Zusatz von Schwefelsäure gezeigt. Werden Verbindungen der Formel VIII, worin R² ein abhydrierbarer Rest, wie Benzyl ist, in die Hydrierungsreaktion eingesetzt, entstehen Verbindungen der allgemeinen Formel I mit R²= Wasserstoff.

Besonders hervorzuheben in den erfindungsgemäßen Verfahren ist die Selektivität der Hydrierungsreaktion, die unter den oben angegebenen Bedingungen diastereoselektiv das cis-, endo-Produkt ergibt.

Die folgenden Beispiele dienen zur Erläuterung der Erfindung.

### Beispiel 1

### 1-Benzyl-1,4,5,6-tetrahydro-cyclopenta[b]pyrrol-2-carbonsäureethylester

46,7 g (0,358 Mol) 2-Chlor-1-formyl-cyclopenten-1 werden in 400 ml Toluol gelöst. Dazu werden 138 g (0,716 Mol) N-Benzylglycinethylester zugeben. Es wird eine Stunde refluxiert, wobei das Reaktionswasser über einen Wasserabscheider abgeschieden wird. Nach dem Abkühlen wird evtl. entstandener Niederschlag abgesaugt, die Toluollösung mit Wasser gewaschen und nach dem Trocknen eingeengt. Der Rückstand wird in 300 ml Ethanol aufgenommen, mit 600 ml 2n-Salzsäure versetzt und 3x mit Petrolether extrahiert. Die Petroletherphase wird 2x mit je 175 ml Ethanol/2n-Salzsäure 1 : 2, danach mit 100 ml gesättigte NaHCO₃-Lösung gewaschen, die organische Phase über MgSO₄ getrocknet, mit Aktivkohle verrührt, abgesaugt und im Vakuum eingedampft.
Ausbeute: 53.1 g Öl
¹H-NMR (CDCl₃, 60 MHz) : δ= 1,26 (t; CH₃), 2,2 - 2,8 (m, 6H, 3CH₂), 4, 18 (q; O-CH₂), 5,48 (S; N-CH₂), 6,78 (S, Pyrrol-H), 6,9 - 7,5 (m; C₆H₅)

### Beispiel 2

### 1-Benzyl-1,4,5,6-tetrahydro-cyclopenta[b]pyrrol-2-carbonsäureethylester

25 g (0,191 Mol) 2-Chlor-1-formyl-cyclopenten-1 werden bei Raumtemperatur zu 73.9 g (0,382 Mol) N-Benzylglycinethylester gegeben und anschließend auf 110°C erhitzt. Die Aufarbeitung erfolgt wie im Beispiel 1 angegeben.

### Beispiel 3

### cis,endo-2-Azabicylco[3,3,0]octan-3-carbonsäureethylester

53,1 g 1-Benzyl-1,4,5,6-tetrahydro-cyclopenta[b]pyrrol-2-carbonsäureethylester werden in 450 ml Ethanol gelöst und zu dieser Lösung 5 g Pd/C (10 %ig) und 10,5 ml (19,3 g) konzentrierter Schwefelsäure gegeben. Anschließend wird bei 30°C und 100 bar Wasserstoffdruck 24 Std. hydriert. Nach dem Absaugen des Katalysators wird die Lösung zur Hälfte im Vakuum eingeengt und mit 2n-Natronlauge auf pH 7 eingestellt. Nach dem Einengen am Rotavapor® wird der Rückstand in 2n Salzsäure aufgenommen und die wäßrige Lösung mit Methylenchlorid gewaschen. Anschließend wird mit K₂CO₃ basisch gestellt, mit NaCl gesättigt und mit Methylendichlorid extrahiert. Nach dem Trocknen über MgSO₄ und Behandlung mit Aktivkohle wird im Vakuum eingeengt.
Ausbeute 28 g
¹H-NMR (CDCl₃, 270 MHz): 1,25 (t; 3H), 1,32 - 1,70 (m, 7 H) 1,32 (breites s; NH) 2,27 - 2,39 (m; 1H), 2,50 - 2,66 (m; 1H) 3,59 (dd J₁= 6 Hz, J₂= 10 Hz; C₃-H); 3,63 (m; C₁-H), 4,17 (q,OCH₂)

### Beispiel 4

### 2-Methyl-cis,endo-2-azabicyclo[3,3,0]octan-3-carbonsäureethylester

1,0 g 1-Methyl-1,4,5,6-tetrahydro-cyclopenta[b]pyrrol-2-carbonsäureethylester (S. Hauptmann et al., J. Prakt. Chem. 314, 353 (1972)) werden in 50 ml absolutem Alkohol gelöst und mit 1,6 ml konz. Schwefelsäure versetzt. Dazu werden 200 mg Rhodium auf Kohle (5 %) gegeben und 24 Std. bei 30°C bei 10 bar Wasserstoffdruck hydriert. Nach dem Absaugen des Katalysators werden 8,3 g gesättigte K₂CO₃-Lösung zugegeben; die Lösung wird mit Methylenchlorid extrahiert, über MgSO₄ getrocknet und eingeengt.
Ausbeute 1,0 g (97 % d. Th.) Öl, das lt. 270 MHz ¹H-NMR einheitlich ist (cis,endo-Konfiguration)
¹H-NMR (CDCl₃, 270 MHz): 1,26 (t, CH₃), 1,30 - 1,81 (m, 7H), 2,19 - 2,31 (m, C₄-H überlappend mit s 2,30, N-CH₃), 2,44 - 2,60 (m; 1H), 2,78 (dd, J₁= 6 Hz, J₂= 8 Hz; C₁-H), 2,92 (dd, J₁= 6 Hz, J₂= 12 Hz; C₃-H), 4,17 (q, OCH₂)

## Patentansprüche (Patentansprüche für folgende(n) Vertragsstaat(en): BE, CH, DE, FR, GB, IT, LI, LU, NL, SE)

1. Verbindung der Formel I, 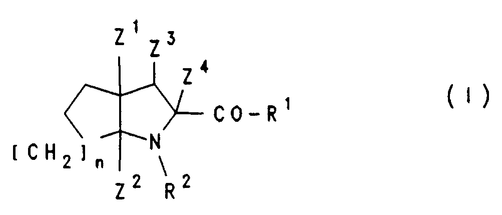 in welcher
n = 1, 2 oder 3 ist und
Z¹ und Z² sowie Z³ und Z⁴ jeweils zusammen für eine chemische Bindung stehen,
R¹ (C₁-C₆)-Alkoxy, (C₆-C₁₂)-Aryl-(C₁-C₄)-alkoxy, Amino, (C₁-C₆)-Alkylamino oder Di-(C₁-C₆)-alkylamino und
R² Wasserstoff, (C₁-C₈)-Alkyl, (C₆-C₁₂)-Aryl, (C₆-C₁₂)-Aryl-(C₁-C₆)-alkyl, p-Nitrobenzyl, p-Methoxybenzyl, (C₃-C₈)-Cycloalkyl oder (C₇-C₁₂)-Alkylcycloalkyl bedeuten,
wobei Verbindungen der Formel I ausgenommen sind, worin
a)
n = 1, 2 oder 3,
R¹ Ethoxy und
R² Methyl bedeuten, oder
b)
n = 1 bedeutet,
R¹ wie oben definiert ist und
R² Benzyl bedeutet.

2. Verbindung gemäß Anspruch 1, dadurch gekennzeichnet, daß
R¹ (C₁-C₆)-Alkoxy und
R² Wasserstoff, (C₆-C₁₂)-Aryl-(C₁-C₆)-alkyl,
p-Nitrobenzyl oder p-Methoxybenzyl bedeuten.

3. Verfahren zur Herstellung einer Verbindung der Formel I,
in welcher
n = 1, 2 oder 3 ist,
Z¹, Z², Z³ und Z⁴ jeweils für Wasserstoff stehen, wobei
Z¹ und Z² zueinander cis-konfiguriert sind und die COR¹-Gruppe am C-Atom 3 endoständig zum bicyclischen Ringsystem orientiert ist,
R¹ (C₁-C₆)-Alkoxy, (C₆-C₁₂)-Aryl-(C₁-C₄)-alkoxy, Amino, (C₁-C₆)-Alkylamino oder Di-(C₁-C₆)-alkylamino und
R² Wasserstoff, (C₁-C₈)-Alkyl, (C₆-C₁₂)-Aryl, (C₆-C₁₂)-Aryl-(C₁-C₆)-alkyl, p-Nitrobenzyl, p-Methoxybenzyl, (C₃-C₈)-Cycloalkyl oder (C₇-C₁₂)-Alkylcycloalkyl bedeuten,
dadurch gekennzeichnet, daß man eine Verbindung der Formel I, in welcher
Z¹ und Z² sowie Z³ und Z⁴ jeweils zusammen für eine
chemische Bindung stehen und
n, R¹ und R² wie oben definiert sind,
ausgenommen Verbindungen der Formel I, worin
n = 1 bedeutet,
R¹ wie oben definiert ist und
R² Benzyl bedeutet,
ausgehend von einem
Glycinderivat der Formel II
R²-NH-CH₂-CO-R¹ (II)
in welcher R¹ und R² wie in Anspruch 1 definiert sind, und einer Verbindung der Formel III 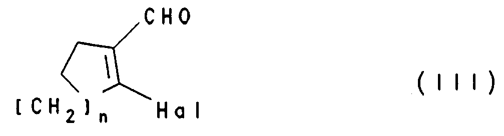 in welcher n = 1, 2 oder 3 ist und Hal für Halogen vorzugsweise Chlor steht, herstellt
und diese dann katalytisch hydriert.

4. Verfahren gemäß Anspruch 3 bei dem Verbindungen hergestellt werden, worin
R¹ (C₁-C₆)-Alkoxy und
R² Wasserstoff, (C₆-C₁₂)-Aryl-(C₁-C₆)-alkyl, p-Nitrobenzyl oder p-Methoxybenzyl bedeuten.

5. Verfahren zur Herstellung einer Verbindung gemäß Anspruch 1, dadurch gekennzeichnet, daß man ein Glycinderivat der Formel II
R²-NH-CH₂-CO-R¹ (II)
in welcher R¹ und R² wie in Anspruch 1 definiert sind, umsetzt mit einer Verbindung der Formel III 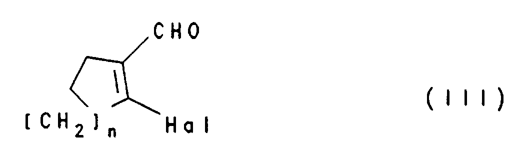 in welcher n = 1, 2 oder 3 ist und Hal für Halogen, vorzugsweise Chlor, steht.

6. Verfahren gemäß Anspruch 4, dadurch gekennzeichnet, daß Verbindungen hergestellt werden, worin das C-Atom in Position 3 des Bicyclus S-konfiguriert ist.

7. Verwendung von Verbindungen hergestellt gemäß Anspruch 4 zur Herstellung von Inhibitoren des Angiotensin Converting Enzyms.

## Patentansprüche (Patentansprüche für folgende(n) Vertragsstaat(en): AT)

1. Verfahren zur Herstellung einer Verbindung der Formel I, 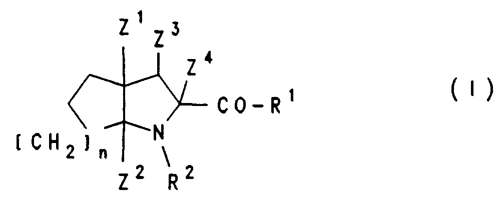 in welcher
n = 1, 2 oder 3 ist und
Z¹ und Z² sowie Z³ und Z⁴ jeweils zusammen für eine chemische Bindung stehen,
R¹ (C₁-C₆)-Alkoxy, (C₆-C₁₂)-Aryl-(C₁-C₄)-alkoxy, Amino, (C₁-C₆)-Alkylamino oder Di-(C₁-C₆)-alkylamino und
R² Wasserstoff, (C₁-C₈)Alkyl, (C₆-C₁₂)-Aryl, (C₆-C₁₂)-Aryl-(C₁-C₆)-alkyl, p-Nitrobenzyl, p-Methoxybenzyl, (C₃-C₈)-Cycloalkyl oder (C₇-C₁₂)-Alkylcycloalkyl bedeuten,
wobei Verbindungen der Formel I ausgenommen sind, worin
a)
n = 1, 2 oder 3,
R¹ Ethoxy und
R² Methyl bedeuten, oder
b)
n = 1 bedeutet,
R¹ wie oben definiert ist und
R² Benzyl bedeutet,
dadurch gekennzeichnet, daß man ein Glycinderivat der Formel II
R²-NH-CH₂-CO-R¹ (II)
in welcher R¹ und R² wie oben definiert sind, umsetzt mit einer Verbindung der Formel III 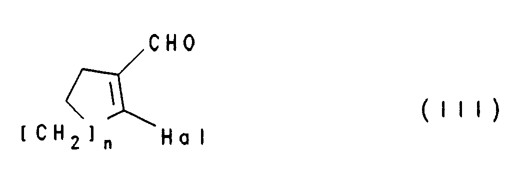 in welcher n = 1, 2 oder 3 ist und Hal für Halogen, vorzugsweise Chlor, steht.

2. Verfahren zur Herstellung einer Verbindung gemäß Anspruch 1, in welcher
R¹ (C₁-C₆)-Alkoxy und
R² Wasserstoff, (C₆-C₁₂)-Aryl-(C₁-C₆)-alkyl,
p-Nitrobenzyl oder p-Methoxybenzyl bedeuten.

3. Verfahren zur Herstellung einer Verbindung der Formel I,
in welcher
n = 1, 2 oder 3 ist,
Z¹, Z², Z³ und Z⁴ jeweils für Wasserstoff stehen, wobei
Z¹ und Z² zueinander cis-konfiguriert sind und die COR¹-Gruppe am C-Atom 3 endoständig zum bicyclischen Ringsystem orientiert ist,
R¹ (C₁-C₆)-Alkoxy, (C₆-C₁₂)-Aryl-(C₁-C₄)-alkoxy, Amino, (C₁-C₆)-Alkylamino oder Di-(C₁-C₆)-alkylamino und
R² Wasserstoff, (C₁-C₈)-Alkyl, (C₆-C₁₂)-Aryl, (C₆-C₁₂)-Aryl-(C₁-C₆)-alkyl, p-Nitrobenzyl, p-Methoxybenzyl, (C₃-C₈)-Cycloalkyl oder (C₇-C₁₂)-Alkylcycloalkyl bedeuten,
dadurch gekennzeichnet, daß man eine Verbindung der Formel I, in welcher
Z¹ und Z² sowie Z³ und Z⁴ jeweils zusammen für eine chemische Bindung stehen und
n, R¹ und R² wie oben definiert sind,
ausgenommen Verbindungen der Formel I, worin
n = 1 bedeutet,
R¹ wie oben definiert ist und
R² Benzyl bedeutet,
ausgehend von einem
Glycinderivat der Formel II
R²-NH-CH₂-CO-R¹ (II)
in welcher R¹ und R² wie in Anspruch 1 definiert sind, und einer Verbindung der Formel III 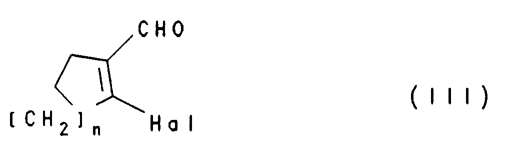 in welcher n = 1, 2 oder 3 ist und Hal für Halogen, vorzugsweise Chlor steht, herstellt
und diese dann katalytisch hydriert.

4. Verfahren gemäß Anspruch 3 bei dem Verbindungen hergestellt werden, worin
R¹ (C₁-C₆)-Alkoxy und
R² Wasserstoff, (C₆-C₁₂)-Aryl-(C₁-C₆)-alkyl, p-Nitrobenzyl oder p-Methoxybenzyl bedeuten.

5. Verfahren gemäß Anspruch 4, dadurch gekennzeichnet, daß Verbindungen hergestellt werden, worin das C-Atom in Position 3 des Bicyclus S-konfiguriert ist.

6. Verwendung von Verbindungen hergestellt gemäß Anspruch 4 zur Herstellung von Inhibitoren des Angiotensin Converting Enzyms.

## Claims (Claims for the following Contracting State(s): BE, CH, DE, FR, GB, IT, LI, LU, NL, SE)

1. A compound of the formula I 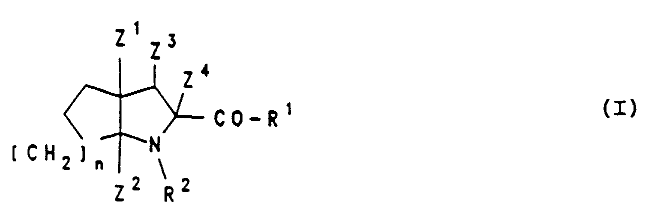 in which
n is 1, 2 or 3 and
Z¹ and Z², and Z³ and Z⁴, in each case together represent a chemical bond,
R¹ denotes (C₁-C₆)-alkoxy, (C₆-C₁₂)-aryl-(C₁-C₄)-alkoxy, amino, (C₁-C₆)-alkylamino or di-(C₁-C₆)-alkylamino and
R² denotes hydrogen, (C₁-C₈)-alkyl, (C₆-C₁₂)-aryl, (C₆-C₁₂)-aryl-(C₁-C₆)-alkyl, p-nitrobenzyl, p-methoxybenzyl, (C₃-C₈)-cycloalkyl or (C₇-C₁₂)-alkylcycloalkyl,
with the exception of compounds of the formula I in which
a)
n is 1, 2 or 3,
R¹ denotes ethoxy and
R² denotes methyl, or
b)
n is 1,
R¹ is defined as above, and
R² denotes benzyl.

2. A compound as claimed in claim 1, wherein
R¹ denotes (C₁-C₆)-alkoxy and
R² denotes hydrogen, (C₆-C₁₂)-aryl-(C₁-C₆)-alkyl, p-nitrobenzyl or p-methoxybenzyl.

3. A process for the preparation of a compound of the formula I in which
n is 1, 2 or 3,
Z¹, Z², Z³ and Z⁴ in each case represent hydrogen,
Z¹ and Z² being in the cis configuration relative to one another and the COR¹ group on C atom 3 being in the endo configuration relative to the bicyclic ring system,
R¹ denotes (C₁-C₆)-alkoxy, (C₆-C₁₂)-aryl-(C₁-C₄)-alkoxy, amino, (C₁-C₆)-alkylamino or di-(C₁-C₆)-alkylamino and
R² denotes hydrogen, (C₁-C₈)-alkyl, (C₆-C₁₂)-aryl, (C₆-C₁₂)-aryl-(C₁-C₆)-alkyl, p-nitrobenzyl, p-methoxybenzyl, (C₃-C₈)-cycloalkyl or (C₇-C₁₂)-alkylcycloalkyl,
wherein a compound of the formula I in which Z¹ and Z², and Z³ and Z⁴, in each case together represent a chemical bond and
n, R¹ and R² are defined as above,
with the exception of compounds of the formula I in which
n is 1,
R¹ is defined as above, and
R² denotes benzyl,
is prepared starting from a
glycine derivative of the formula II
R²-NH-CH₂-CO-R¹ (II)
in which R¹ and R² are defined as in claim 1, and from a compound of the formula III 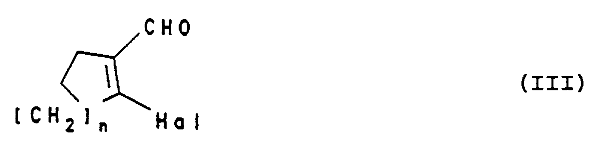 in which n is 1, 2 or 3 and Hal represents halogen, preferably chlorine, and is then catalytically hydrogenated.

4. The process as claimed in claim 3, wherein compounds are prepared in which
R¹ denotes (C₁-C₆)-alkoxy and
R² denotes hydrogen, (C₆-C₁₂)-aryl-(C₁-C₆)-alkyl, p-nitrobenzyl or p-methoxybenzyl.

5. A process for the preparation of a compound as claimed in claim 1, which comprises reacting a glycine derivative of the formula II
R²-NH-CH₂-CO-R¹ (II)
in which R¹ and R² are defined as in claim 1 with a compound of the formula III 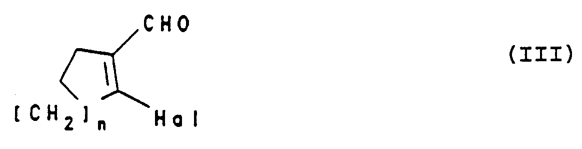 in which n is 1, 2 or 3 and Hal represents halogen, preferably chlorine.

6. The process as claimed in claim 4, which comprises preparing compounds in which the C atom in position 3 of the bicyclic ring system is in the S configuration.

7. The use of compounds prepared as claimed in claim 4 for the preparation of angiotensin-converting enzyme inhibitors.

## Claims (Claims for the following Contracting State(s): AT)

1. A process for the preparation of a compound of the formula I 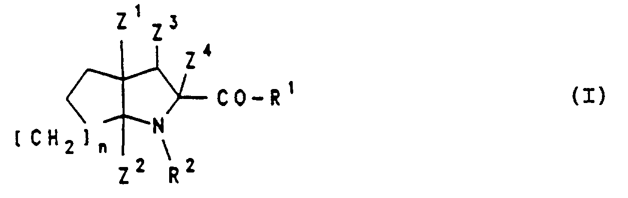 in which
n is 1, 2 or 3 and
Z¹ and Z², and Z³ and Z⁴, in each case together represent a chemical bond,
R¹ denotes (C₁-C₆)-alkoxy, (C₆-C₁₂)-aryl-(C₁-C₄)-alkoxy, amino, (C₁-C₆)-alkylamino or di-(C₁-C₆)-alkylamino and
R² denotes hydrogen, (C₁-C₈)-alkyl, (C₆-C₁₂)-aryl, (C₆-C₁₂)-aryl-(C₁-C₆)-alkyl, p-nitrobenzyl, p-methoxybenzyl, (C₃-C₈)-cycloalkyl or (C₇-C₁₂)-alkylcycloalkyl,
with the exception of compounds of the formula I in which
a)
n is 1, 2 or 3,
R¹ denotes ethoxy and
R² denotes methyl, or
b)
n is 1,
R¹ is defined as above, and
R² denotes benzyl,
which comprises reacting a glycine derivative of the formula II
R²-NH-CH₂-CO-R¹ (II)
in which R¹ and R² are defined as above with a compound of the formula III 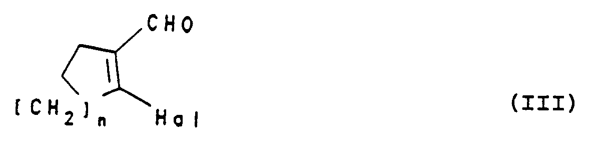 in which n is 1, 2 or 3 and Hal represents halogen, preferably chlorine.

2. A process for the preparation of a compound as claimed in claim 1, wherein
R¹ denotes (C₁-C₆)-alkoxy and
R² denotes hydrogen, (C₆-C₁₂)-aryl-(C₁-C₆)-alkyl,
p-nitrobenzyl or p-methoxybenzyl.

3. A process for the preparation of a compound of the formula I in which
n is 1, 2 or 3,
Z¹, Z², Z³ and Z⁴ in each case represent hydrogen,
Z¹ and Z² being in the cis configuration relative to one another and the COR¹ group on C atom 3 being in the endo configuration relative to the bicyclic ring system,
R¹ denotes (C₁-C₆)-alkoxy, (C₆-C₁₂)-aryl-(C₁-C₄)-alkoxy, amino, (C₁-C₆)-alkylamino or di-(C₁-C₆)-alkylamino and
R² denotes hydrogen, (C₁-C₈)-alkyl, (C₆-C₁₂)-aryl, (C₆-C₁₂)-aryl-(C₁-C₆)-alkyl, p-nitrobenzyl, p-methoxybenzyl, (C₃-C₈)-cycloalkyl or (C₇-C₁₂)-alkylcycloalkyl,
wherein a compound of the formula I in which Z¹ and Z², and Z³ and Z⁴, in each case together represent a chemical bond and
n, R¹ and R² are defined as above,
with the exception of compounds of the formula I in which
n is 1,
R¹ is defined as above, and
R² denotes benzyl,
is prepared starting from a
glycine derivative of the formula II
R²-NH-CH₂-CO-R¹ (II)
in which R¹ and R² are defined as in claim 1, and from a compound of the formula III 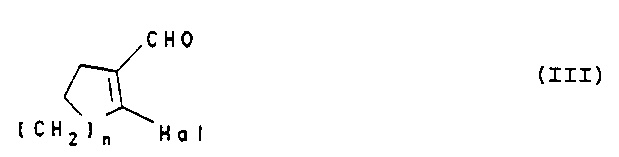 in which n is 1, 2 or 3 and Hal represents halogen, preferably chlorine, and is then catalytically hydrogenated.

4. The process as claimed in claim 3, wherein compounds are prepared in which
R¹ denotes (C₁-C₆)-alkoxy and
R² denotes hydrogen, (C₆-C₁₂)-aryl-(C₁-C₆)-alkyl, p-nitrobenzyl or p-methoxybenzyl.

5. The process as claimed in claim 4, which comprises preparing compounds in which the C atom in position 3 of the bicyclic ring system is in the S configuration.

6. The use of compounds prepared as claimed in claim 4 for the preparation of angiotensin-converting enzyme inhibitors.

## Revendications (Revendications pour l'(les) Etat(s) contractant(s) suivant(s): BE, CH, DE, FR, GB, IT, LI, LU, NL, SE)

1. Composé de formule I 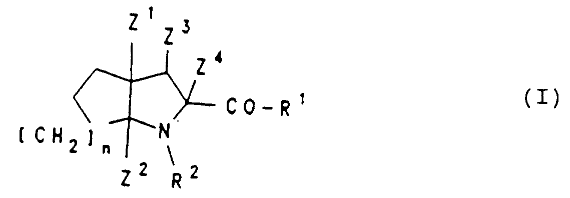 dans laquelle
n est 1, 2 ou 3, et
Z¹ et Z² ainsi que Z³ et Z ⁴ représentent ensemble, respectivement, une liaison chimique,
R¹ représente un groupe alcoxy en C₁-C₆, aryl(C₆-C₁₂)-alcoxy(C₁-C₄), amino, alkyl(C₁-C₆)-amino ou dialkyl(C₁-C₆)-amino, et
R² représente un atome d'hydrogène ou un groupe alkyle en C₁-C₈, aryle en C₆-C₁₂, aryl(C₆-C₁₂)-alkyle(C₁-C₆), p-nitrobenzyle, p-méthoxybenzyle, cycloalkyle en C₃-C₈ ou alkylcycloalkyle en C₇-C₁₂
à l'exclusion des composés de formule I dans lesquels
a)
n = 1, 2 ou 3,
R¹ représente le groupe éthoxy et
R² représente le groupe méthyle, ou
b)
n = 1,
R¹ est tel que défini plus haut et
R² représente le groupe benzyle.

2. Composé selon la revendication 1, caractérisé en ce que
R¹ représente un groupe alcoxy en C₁-C₆ et
R² représente un atome d'hydrogène ou un groupe aryl(C₆-C₁₂)-alkyle(C₁-C₆), p-nitrobenzyle ou p-méthoxybenzyle.

3. Procédé pour la préparation d'un composé de formule I dans laquelle
n = 1, 2 ou 3,
Z¹, Z², Z³ et Z⁴ représentent chacun un atome d'hydrogène,
Z¹ et Z² étant en configuration *cis* l'un par rapport à l'autre et le groupe COR¹ sur l'atome de carbone 3 étant en orientation endo par rapport au système bicyclique,
R¹ représente un groupe alcoxy en C₁-C₆, aryl(C₆-C₁₂)-alcoxy(C₁-C₄), amino, alkyl(C₁-C₆)-amino ou dialkyl(C₁-C₆)-amino et
R² représente un atome d'hydrogène ou un groupe alkyle en C₁-C₈, aryle en C₆-C₁₂, aryl(C₆-C₁₂)-alkyle(C₁-C₆), p-nitrobenzyle, p-méthoxybenzyle, cycloalkyle en C₃-C₈ ou alkylcycloalkyle en C₇-C₁₂,
caractérisé en ce que l'on prépare un composé de formule I dans lequel
Z¹ et Z² ainsi que Z³ et Z⁴ représentent ensemble, respectivement, une liaison chimique, et,
n, R¹ et R² sont tels que définis plus haut,
à l'exclusion des composés de formule I dans lesquels
n = 1,
R¹ est tel que défini plus haut et
R² représente le groupe benzyle,
à partir d'un dérivé de glycine de formule II
R²-NH-CH₂-CO-R¹ (II)
dans laquelle R¹ et R² sont tels que définis dans la revendication 1, et d'un composé de formule III 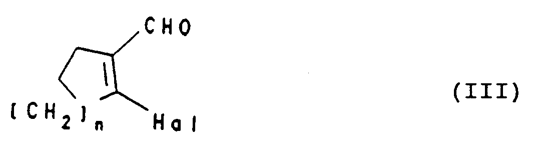 dans laquelle n = 1, 2 ou 3 et Hal représente un atome d'halogène, de préférence de chlore,
et on le soumet ensuite à une hydrogénation catalytique.

4. Procédé selon la revendication 3, dans lequel on prépare des composés dans lesquels
R¹ représente un groupe alcoxy en C₁-C₆ et
R² représente un atome d'hydrogène ou un groupe aryl(C₆-C₁₂)-alkyle(C₁-C₆), p-nitrobenzyle ou p-méthoxybenzyle.

5. Procédé pour la préparation d'un composé selon la revendication 1, caractérisé en ce que l'on fait réagir un dérivé de glycine de formule II
R²-NH-CH₂-CO-R¹ (II)
dans laquelle R¹ et R² sont tels que définis dans la revendication 1, avec un composé de formule III 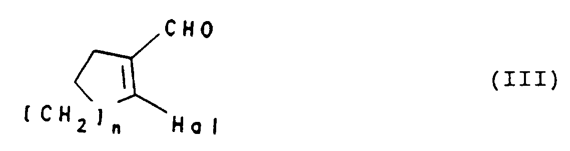 dans laquelle n = 1, 2 ou 3 et Hal représente un atome d'halogène, de préférence de chlore.

6. Procédé selon la revendication 4, caractérisé en ce que l'on prépare des composés dans lesquels l'atome de carbone en position 3 du système bicyclique a la configuration S.

7. Utilisation de composés préparés selon la revendication 4, pour la préparation d'inhibiteurs de l'enzyme de conversion de l'angiotensine.

## Revendications (Revendications pour l'(les) Etat(s) contractant(s) suivant(s): AT)

1. Procédé pour la préparation d'un composé de formule I 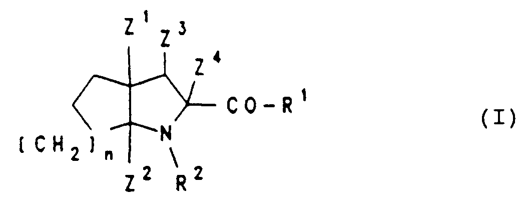 dans laquelle
n est 1, 2 ou 3, et
Z¹ et Z² ainsi que Z³ et Z ⁴ représentent ensemble, respectivement, une liaison chimique,
R¹ représente un groupe alcoxy en C₁-C₆, aryl(C₆-C₁₂)-alcoxy(C₁-C₄), amino, alkyl(C₁-C₆)-amino ou dialkyl(C₁-C₆)-amino, et
R² représente un atome d'hydrogène ou un groupe alkyle en C₁-C₈, aryle en C₆-C₁₂, aryl(C₆-C₁₂)-alkyle(C₁-C₆), p-nitrobenzyle, p-méthoxybenzyle, cycloalkyle en C₃-C₈ ou alkylcycloalkyle en C₇-C₁₂
à l'exclusion des composés de formule I dans lesquels
a)
n = 1, 2 ou 3,
R¹ représente le groupe éthoxy et
R² représente le groupe méthyle, ou
b)
n = 1,
R¹ est tel que défini plus haut et
R² représente le groupe benzyle,
caractérisé en ce que l'on fait réagir un dérivé de glycine de formule II
R²-NH-CH₂-CO-R¹ (II)
dans laquelle R¹ et R² sont tels que définis plus haut, avec un composé de formule III 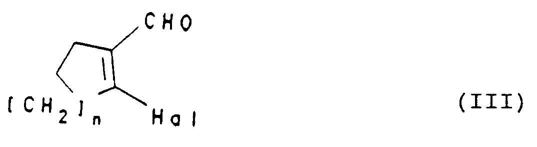 dans laquelle n = 1, 2 ou 3 et Hal représente un atome d'halogène, de préférence de chlore.

2. Procédé pour la préparation d'un composé selon la revendication 1, dans lequel
R¹ représente un groupe alcoxy en C₁-C₆ et
R² représente un atome d'hydrogène ou un groupe aryl(C₆-C₁₂)-alkyle(C₁-C₆), p-nitrobenzyle ou p-méthoxybenzyle.

3. Procédé pour la préparation d'un composé de formule I, dans lequel
n = 1, 2 ou 3,
Z¹, Z², Z³ et Z⁴ représentent chacun un atome d'hydrogène,
Z¹ et Z² étant en configuration cis l'un par rapport à l'autre et le groupe COR¹ sur l'atome de carbone 3 étant en orientation endo par rapport au système bicyclique,
R¹ représente un groupe alcoxy en C₁-C₆, aryl(C₆-C₁₂)-alcoxy(C₁-C₄), amino, alkyl(C₁-C₆)-amino ou dialkyl(C₁-C₆)-amino et
R² représente un atome d'hydrogène ou un groupe alkyle en C₁-C₈, aryle en C₆-C₁₂, aryl(C₆-C₁₂)-alkyle(C₁-C₆), p-nitrobenzyle, p-méthoxybenzyle, cycloalkyle en C₃-C₈ ou alkylcycloalkyle en C₇-C₁₂,
caractérisé en ce que l'on prépare un composé de formule I dans lequel
Z¹ et Z² ainsi que Z³ et Z⁴ représentent ensemble, respectivement, une liaison chimique, et,
n, R¹ et R² sont tels que définis plus haut,
à l'exclusion des composés de formule I dans lesquels
n = 1,
R¹ est tel que défini plus haut et
R² représente le groupe benzyle,
à partir d'un dérivé de glycine de formule II
R²-NH-CH₂-CO-R¹ (II)
dans laquelle R¹ et R² sont tels que définis dans la revendication 1 et d'un composé de formule III 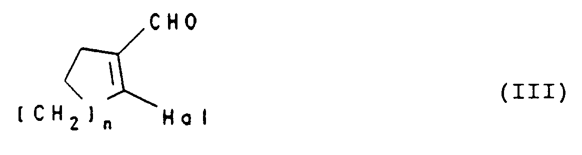 dans laquelle n = 1, 2 ou 3 et Hal représente un atome d'halogène, de préférence de chlore,
et on le soumet ensuite à une hydrogénation catalytique.

4. Procédé selon la revendication 3, dans lequel on prépare des composés dans lesquels
R¹ représente un groupe alcoxy en C₁-C₆ et
R² représente un atome d'hydrogène ou un groupe aryl(C₆-C₁₂)-alkyle(C₁-C₆), p-nitrobenzyle ou p-méthoxybenzyle.

5. Procédé selon la revendication 4, caractérisé en ce que l'on prépare des composés dans lesquels l'atome de carbone en position 3 du système bicyclique a la configuration S.

6. Utilisation de composés préparés selon la revendication 4, pour la préparation d'inhibiteurs de l'enzyme de conversion de l'angiotensine.
